(19) Europäisches Patentamt
European Patent Office
Office européen des brevets

(11) Veröffentlichungsnummer: **0 055 797**
**B1**

(12) # EUROPÄISCHE PATENTSCHRIFT

(45) Veröffentlichungstag der Patentschrift: **29.05.85**

(51) Int. Cl.⁴: **C 07 C 51/38**, C 07 C 67/32, C 07 C 120/00

(21) Anmeldenummer: **81107974.8**

(22) Anmeldetag: **06.10.81**

(54) **Verfahren zur Herstellung substituierter Essigsäuren und/oder deren Estern bzw. substituierter Acetonitrile.**

(30) Priorität: **29.11.80 DE 3045102**

(43) Veröffentlichungstag der Anmeldung: **14.07.82 Patentblatt 82/28**

(45) Bekanntmachung des Hinweises auf die Patenterteilung: **29.05.85 Patentblatt 85/22**

(84) Benannte Vertragsstaaten: **BE DE FR GB NL**

(56) Entgegenhaltungen:
**US - A - 1 953 232**
**US - A - 1 956 718**
**US - A - 3 476 803**

**CHEMISCHES ZENTRALBLATT, Band 137, Nr. 40, 1966, Berlin W.J. BAILEY et al. "Pyrolyse von Estern. 25. Mitt. Pyrolyse von Malonsäureestern" Referat Nr. 0856**
**Patents Abstracts of Japan Band 3, Nr. 142, 24. November 1979 Seite 115C65**
**Patents Abstracts of Japan Band 4, Nr. 173, 29. November 1980 Seite 655C32**

**Die Akte enthält technische Angaben, die nach dem Eingang der Anmeldung eingereicht wurden und die nicht in dieser Patentschrift enthalten sind.**

(73) Patentinhaber: **DYNAMIT NOBEL AKTIENGESELLSCHAFT, Postfach 1209, D-5210 Troisdorf, Bez. Köln (DE)**

(72) Erfinder: **Ackermann, Otto, Dr., Uhlandstrasse 4, D-5210 Troisdorf (DE)**
Erfinder: **Daum, Gerhard, Dr., Erkerstrasse 15c, D-5000 Köln 90 (DE)**

**EP 0 055 797 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein neues allgemeines Verfahren zur Herstellung substituierter Essigsäuren sowie deren Derivate wie z. B. die Alkali- oder Erdalkali-Salze der substituierten Essigsäuren und/oder der Ester bzw. substituierter Acetonitrile.

Aus der großen Zahl der nach dem erfindungsgemäßen Verfahren zugänglichen Verbindungen seien die Homologen der Essigsäuren wie Propionsäure, Buttersäure oder Valeriansäure sowie deren Nitrile genannt. Diese durch unverzweigte aliphatische Reste monosubstituierten Essigsäuren sind jedoch in der Regel weniger bevorzugt als die verzweigtkettigen Verbindungen und arylsubstituierten Essigsäuren oder Acetonitrile.

Sehr bevorzugt hergestellt werden erfindungsgemäß bisubstituierte Essigsäuren oder Acetonitrile.

Beispiele für solche Verbindungen sind 2-Phenylpropionsäure aus Phenyl-methylmalonsäureestern, 2-Phenylbuttersäure aus Phenyl-äthylmalonsäureestern, 2-Phenylvaleriansäure aus Phenyl-propylmalonsäureestern, Phenyl-cycloalkylessigsäuren aus Phenyl-cycloalkylmalonestern sowie die durch zwei aliphatische Reste substituierten Essigsäuren wie 2-Methylpropionsäure, 2-Methylbuttersäure, 2-Methylvaleriansäure, 2-Methylcapronsäure und Dipropylessigsäure, die auch 2-Propylvaleriansäure heißt. Dipropylessigsäure ist in Form ihrer Salze ein bekanntes Pharmakon und ist daher als Säure und/oder Ester sehr bevorzugtes Zielprodukt des Verfahrens.

Die Herstellung einer Anzahl substituierter Essigsäureester aus entsprechend substituierten Malonsäureestern durch Pyrolyse in Abwesenheit von Katalysatoren war bekannt.

So wird nach W. J. Bailey und J. J. Daly, J. Org. Chem. 29 (5) 1964, 1249-51, durch Pyrolyse von Phenylmalonester Phenylessigsäure und von Methylbutylmalonester 3-Methylcapronsäure gewonnen. Trotz hoher Temperaturen zwischen 457 und 560°C wird nur ein Teil der Substanz umgesetzt und von dieser ein Teil zum Zielprodukt umgewandelt.

In der Folgezeit wurden daher zur Herstellung von Dipropylessigsäure vielstufige Reaktionen ausgehend von Buttersäure nach DE-OS 2 844 638 oder n-Valeraldehyd-diallylacetal nach DE-OS 2 844 636 vorgeschlagen, deren Verfahrenswege kompliziert und deren Ausbeuten ebenfalls nicht hoch sind.

Nach der DE-OS 2 853 732 wird aus Malonsäurediäthylester der Dipropylmalonester gebildet, dann durch Ansäuern zunächst die reine Dipropylmalonsäure isoliert, gereinigt und getrocknet und erst dann bei Temperaturen von 160—165°C, d. h. oberhalb des Schmelzpunktes zu Dipropylessigsäure decarboxyliert, die durch fraktionierte Destillation gereinigt werden muß. Die Isolierung der reinen Dipropylmalonsäure stellt eine zusätzliche Verfahrensstufe mit hohen Verlusten dar, so daß bezogen auf den eingesetzten Malonsäurediäthylester nur eine Ausbeute von 42,9% erreicht wird.

Bei diesen Verfahren der Verseifung und Decarboxylierung von Dipropylmalonester entstehen pro Mol Malonester 2 Mol Salz, das abgetrennt und entsorgt werden muß. Die Überführung in Dipropylessigsäure erfordert hohen apparativen, stofflichen und zeitlichen Aufwand, da der Malonester alkalisch verseift wird und das Alkalisalz durch Ansäuern in einer gesonderten Apparatur in die Säure überführt werden muß.

Bei Herstellung aus Dipropylcyanessigester muß nach der Verseifung und Decarboxylierung der Estergruppe zusätzlich die Nitrilgruppe hydrolysiert werden (DE-OS 2 721 264).

Es war aus der JP-A-55-111 443 bekannt, Ester substituierter Malonsäuren in Gegenwart von Katalysatoren aus hydratisiertem Dimethylsulfoxid und Alkalimetallhalogeniden bzw. -cyaniden in die Ester der entsprechend substituierten Essigsäuren zu überführen.

Weiter war aus der US-PS 1 956 718 und der US-PS 3 476 803 die Decarboxylierung von Mono-, Di- und Polycarbonsäuren mit verschiedenen Katalysatoren bekannt, wobei jedoch geringe Ausbeuten der Zielprodukte erhalten werden.

Es wurde nun gefunden, daß einfach und in hoher Ausbeute ein- oder zweifach substituierte Malonester ohne vorherige Bildung der Malonsäuren bzw. ein- oder zweifach substituierte Cyanessigsäureester unter Verwendung von Katalysatoren in die entsprechend substituierten Essigsäureester bzw. Acetonitrile überführt werden können.

Gegenstand der Erfindung ist daher ein Verfahren zur Herstellung substituierter Essigsäuren und/oder deren Ester bzw. substituierter Acetonitrile der Formel I

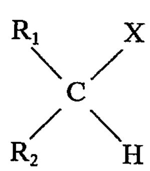

worin X —COOH, —COOY oder —CN, Y —$CH_3$ oder —$C_2H_5$, $R_1$ einen gesättigten, verzweigten oder unverzweigten aliphatischen Rest mit 1 bis 6 C-Atomen, einen Phenylrest oder einen durch Alkyl- oder Alkoxygruppen substituierten Phenylrest und $R_2$ Wasserstoff oder $R_1$ bedeuten, wobei $R_1$ und $R_2$ gleich oder verschieden sein können, aus den entsprechend substituierten Malon- oder Cyanessigestern der Formel (II)

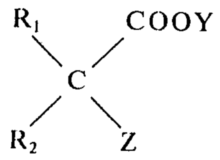

worin Z —COOY oder —CN bedeuten und Y, $R_1$ und $R_2$ die vorige Bedeutung haben, welches dadurch gekennzeichnet ist, daß man die Malon- oder Cyanessigester bei erhöhter Temperatur in Gegenwart von Katalysatoren umsetzt.

Wesentliche Vorteile des Verfahrens sind die Verwendung der leicht zugänglichen substituierten Malonester bzw. substituierten Cyanessigester, deren fast vollständig erreichbare Umsetzung, die fast völlige Vermeidung von Neben- und Zersetzungsprodukten, die vergleichsweise niedrigen Reaktionstemperaturen und die völlige Vermeidung der nach dem Stand der Technik durch Neutralisation entstehenden und abzutrennenden Salze, insgesamt somit erheblich gesenkte Verfahrenskosten.

Sehr bevorzugt wird die Reaktion in Gegenwart von Wasser ausgeführt, wodurch die Erzielung eines vollständigen Umsatzes sehr erleichtert wird. Durch die Gegenwart von Wasser wird der Anteil des je nach Produkt und Reaktionsbedingungen schwankenden Anteils der substituierten Essigsäure im Verhältnis zum substituierten Essigsäureester erhöht. Das ist vorteilhaft besonders im Hinblick auf die wichtigste Weiterverarbeitung der Reaktionsprodukte zu Salzen der substituierten Essigsäuren.

Geeignete Wassermengen liegen zwischen 1 und 50 Mol Wasser, vorzugsweise 10 bis 30 Mol Wasser, pro Mol der Ausgangsstoffe.

Die Reaktion kann auch in Abwesenheit von Wasser ausgeführt werden.

Dabei entstehen substituierte Essigsäureester als Hauptprodukte neben substituierten Essigsäuren.

Auch die Gegenwart eines Alkohols, zweckmäßig des der Estergruppe des substituierten Malonesters entsprechenden Alkohols, ist möglich. Auch dann entstehen kleine Mengen der substituierten Essigsäure neben überwiegenden Mengen der substituierten Essigsäureester. Geeignete Mengen Alkohol liegen ebenfalls zwischen 1 und 50 Mol pro Mol Ausgangsstoff.

Die Reaktion kann auch in Gegenwart von Wasser und dem jeweiligen Alkohol ausgeführt werden.

Die Ausgangsstoffe sind leicht zugängig, beispielsweise aus dem großtechnisch hergestellten Malonester durch Umsetzung mit Alkylhalogeniden bzw. aus dem gleichartig reagierenden Cyanessigester.

Die Reaktionstemperaturen können zwischen 150 und 360°C, vorzugsweise zwischen 180 und 300°C liegen.

Zweckmäßig wird die Temperatur so niedrig wie möglich gehalten, da dann die Bildung von Nebenprodukten und die Substanzzersetzung gering ist. Die anzuwendenden Temperatur ist von der Wirksamkeit des jeweiligen Katalysators abhängig. Die folgend genannten sehr aktiven Katalysatoren bewirken schon bei 150°C geringe Umsätze und haben zwischen 210 und 240°C, besonders zwischen 220 und 230°C ihr Optimum mit sehr hohen Umsätzen und außerordentlich geringen Mengen von Nebenprodukten. Weniger wirksame Katalysatoren benötigten Temperaturen von 300°C und mehr.

Als Reaktor kann ein Rohrreaktor mit Verdampfungszone und Katalysatorzone, zweckmäßig ein senkrecht stehendes, mit dem Katalysator beschicktes und beheiztes Rohr verwendet werden. Die Reaktionspartner werden in die obere Vorheizzone eindosiert, wobei das Wasser verdampft. Der Malonester bzw. Cyanessigester muß nicht vollständig in der Dampfform vorliegen, was einen weiteren positiven Einfluß auf die Reaktion hat, die dadurch bei relativ niedrigen Temperaturen durchgeführt werden kann. Nach dem Durchströmen der Katalysatorzone wird das Reaktionsgemisch gekühlt; es trennt sich dabei in eine wäßrige und eine organische Phase, die anschließend getrennt aufgearbeitet werden. In der wäßrigen Phase ist der freigesetzte Alkohol weitgehend enthalten.

Der Alkohol kann durch Destillation zurückgewonnen werden. Das Wasser oder gegebenenfalls Gemische von Wasser und Alkohol können wieder in den Reaktor eingespeist werden.

Die organische Phase enthält das gewünschte substituierte Acetonitril bzw. die substituierte Essigsäure und den entsprechenden Ester im wechselnden Verhältnis je nach den Reaktionsbedingungen.

Aus der organischen Phase kann der substituierte Essigsäureester von der substituierten Essigsäure durch fraktionierte Destillation einfach getrennt werden, da die Differenz der Siedepunkte groß ist.

Sofern Reste der Ausgangsverbindungen im Produkt enthalten sind, finden diese sich in der organischen Phase. Im Falle der Dipropylessigsäure beispielsweise geht der Ausgangsstoff bei der Destillation mit der Dipropylessigsäure über und ist so vom Dipropylessigsäureester trennbar. Erforderlichenfalls können die Produkte oder die Teilfraktionen ohne Schaden zusammen mit weiteren Ausgangsstoffen wieder zur Reaktion umgesetzt werden. Es ist jedoch der große Fortschritt des vorliegenden katalytischen Pyrolyseverfahrens, daß Gehalte des Ausgangsstoffes im Produkt praktisch völlig vermeidbar sind bei Einhaltung der genannten Temperaturen, Wassermengen sowie bei Wahl der nachfolgend genannten Katalysatoren in für die benötigte Verweilzeit geeigneten Schichthöhen. Die bevorzugten Aufarbeitung erfolgt durch Umsetzung der gesamten organischen Phase zu den Alkali- oder Erdalkalisalze besonders zu Na-, K- oder gegebenenfalls Ca-Salzen ohne Isolierung von Säure und Ester durch Behandlung mit den Hydroxiden in wäßriger Lösung, wobei sich aus der Säure das Salz

sofort bildet und der Ester unter Alkoholabspaltung zum Salz verseift wird.

Als Katalysatoren sind solche von silikatischem und oxidischem Typus mit hohem $SiO_2$-Gehalt oder/und $Al_2O_3$-Gehalt jedoch mit fehlendem Schwermetallgehalt, abgesehen von einem geringen Gehalt von Eisenoxid brauchbar. Im allgemeinen weisen diese Katalysatoren einen Glühverlust, zurückgehend auf Gehalte von Wasser auf.

Die Wirksamkeit dieser Katalysatoren ist jedoch verschieden hoch, in Abhängigkeit von den folgenden Merkmalen.

Hochwirksam und sehr bevorzugt sind solche mit sowohl Gehalten von $SiO_2$ als auch $Al_2O_3$ (Gruppe I), welche einen Glühverlust aufweisen und geringe Gehalten von CaO, MgO und $K_2O$ neben geringen Gehalten von $Na_2O$ aufweisen können. Die am besten wirksame Gruppe von Katalysatoren besitzt Gehalte von etwa 50 bis 75 Gew.-% $SiO_2$ neben etwa 15 bis 19 Gew.-% $Al_2O_2$, 15 bis 20 Gew.-% Glühverlust, 2,5 bis 5,5 Gew.-% verteilt auf CaO, MgO, $K_2O$ neben geringen Gehalten von 0,1 bis 0,4 Gew.-% $Na_2O$ und 2,5 bis 4,0 Gew.-% $Fe_2O_3$.

Die Herstellung dieser am besten wirksamen Katalysatoren kann aus Montmorillonit oder entsprechenden natürlichen Silikaten durch Säurebehandlung, Trocknen und Zubereitung als Katalysatoren erfolgen.

Mit dieser Gruppe von Katalysatoren sind bei den optimalen Temperaturen von 210 bis 240°C insbesondere 220 bis 230°C Umsätze über 99,9% der Ausgangsstoffe erzielbar, jedoch ist die Wirksamkeit der Katalysatoren nicht an diesen Temperaturbereich gebunden.

Soweit feststellbar, ist die hohe Wirksamkeit an den natürlichen Wassergehalt und Aufbau des Silikats, der bei der Säurebehandlung und Aufbereitung ganz oder zum Teil erhalten bleibt, sowie an die genannten Gehalte von $SiO_2$ und $Al_2O_3$ gebunden, während die Gehalte von MgO, CaO, $K_2O$, die geringen Gehalten von $Na_2O$ wie auch die Eisenoxidgehalte durch Herkunft und Zubereitung bedingt sind, aber nicht für die katalytische Wirkung als wesentlich angesehen werden.

Eine andere Gruppe II von Katalysatoren ist weniger wirksam und weniger bevorzugt. Diese Katalysator der Gruppe II können ebenfalls MgO, CaO, $K_2O$, $Na_2O$ und Eisenoxide enthalten und können wie oben angegeben hergestellt werden, weisen aber a) 85—92 Gew.-% $SiO_2$, bis 2 Gew.-% $Al_2O_3$ und Glühverluste von 6,5 bis 8,5 Gew.-% auf oder b) etwa 68—75 Gew.-% $SiI_2$ neben 14 bis 16 Gew.-% $Al_2O_3$ und 6,5 bis 8,5 Gew.-% Glühverlust.

Die Reaktionstemperaturen dieser Gruppe von Katalysatoren sind ab 250°C besonders von 260—290°C optimal, jedoch sind nicht immer Umsätze von 99,9% sondern vielfach nur solche über etwa 98,5% erzielbar und die auf den Umsatz bezogenen Ausbeuten überschreiten nicht immer Werte von etwa 92 bis 94%.

Möglich sind auch Katalysatoren der Gruppe III, die praktisch vollständig aus $Al_2O_3$ bestehen und im Handel als Katalysatorträger vertrieben werden. Die verringerte Wirksamkeit, besonders gegenüber der sehr bevorzugten Gruppe, drückt sich darin aus, daß bei 250—260°C der Umsatz mit 55 bis 65% noch unvollständig ist, aber die Ausbeute bereits 97% vom Umsatz überschreitet, während bei 240 bis 260°C zwar der Umsatz mit über 98% hoch ist, aber dann die Ausbeute durch Zersetzung sinkt.

Die als Katalysatoren verwendeten Stoffe weisen im allgemeinen Korngrößen von 1,5 bis 8 mm auf.

Die Standzeit der Katalysatoren ist gut, so daß eine Regenerierung nur in relativ großen Zeitabständen nötig ist. Bei kontinuierlicher Fahrweise, d. h. im Dauerbetrieb, ist es zweckmäßig, die langsam nachlassende Katalysatoraktivität durch eine geringe Steigerung der Reaktionstemperatur auszugleichen, wobei die Temperatur nur soweit zu steigern ist, daß noch keine nennbare Erhöhung der Zersetzung auftritt.

Erst bei einer stark verminderten Aktivität ist die Regenerierung angebracht. Sie ist in der Praxis ohne jede Schwierigkeit auf die nachstehend beschriebene Weise durchführbar:

Bei 300°C wird etwa die gleiche bis doppelte Gewichtsmenge Wasser, bezogen auf das Gewicht des Katalysators, durch den Reaktor geleitet. Anschließend wird ebenfalls bei ca. 300°C für 2 bis 3 Stunden Luft eingeleitet. Nach dieser Behandlung ist der Katalysator wieder voll wirksam.

Bei den durchgeführten Versuchen ist ein Auswechseln des Katalysators infolge Inaktivität auch nach mehrfachem Regenerieren nicht notwendig gewesen.

Folgende Katalysatoren wurden verwendet (Gehalte in Gew.-%)

| | Glühverlust | $SiO_2$ | $Al_2O_3$ | CaO | MgO | $Fe_2O_3$ | $Na_2O$ | $K_2O$ | Summe |
|---|---|---|---|---|---|---|---|---|---|
| Ia | 18,7 | 58,5 | 15,2 | 0,26 | 1,01 | 2,80 | 0,33 | 1,43 | 98,2 |
| Ib | 13,3 | 55,1 | 18,7 | 1,68 | 2,05 | 3,87 | 0,29 | 1,52 | 96,5 |
| Ic | 16,7 | 55,9 | 18,1 | 1,75 | 2,00 | 3,70 | 0,27 | 1,49 | 99,9 |

(Fortsetzung)

| | Glüh-verlust | $SiO_2$ | $Al_2O_3$ | CaO | MgO | $Fe_2O_3$ | $Na_2O$ | $K_2O$ | Summe |
|---|---|---|---|---|---|---|---|---|---|
| Id | 14,9 | 62,2 | 16,2 | 0,24 | 1,29 | 2,85 | 0,19 | 1,22 | 99,1 |
| Ie | 15,4 | 63,4 | 15,8 | 0,16 | 1,28 | 2,74 | 0,13 | 1,01 | 99,9 |
| IIa | 8,2 | 71,3 | 15,6 | 0,17 | 1,13 | 2,65 | 0,13 | 1,12 | 100,3 |
| IIb | 7,2 | 90,4 | 1,3 | 0,13 | 0,10 | 0,23 | 0,0084 | 0,12 | 99,5 |

## Apparatur

Als Reaktor diente ein senkrecht stehendes, mit dem Katalysator gefülltes, elektrisch beheiztes Quarzrohr von 30 mm Innendurchmesser, auf das ein mit Porzellansattelkörpern gefülltes Verdampfungsrohr vom gleichen Durchmesser aufgesetzt war. Dieses wurde durch einen separaten Heizkreis ebenfalls elektrisch beheizt und war mit je einer Zuleitung für die Ausgangssubstanz und für Wasser oder Alkohol versehen. Am unteren Ende des Reaktors war ein Zweihalskolben mit aufgesetztem Rückflußkühler angebracht, in dem sich das Produkt sammelt. Die Dosierung der Reaktionspartner erfolgte über Membranpumpen, die Abgase wurden aus dem Rückflußkühler über eine Kühlfalle in einen Abzug geleitet. Die Temperaturmessung erfolgte durch Thermofühler, die in einem dünneren Schutzrohr aus Quarz konzentrisch im Reaktor und im Verdampferrohr angebracht waren.

## Beispiel 1

1600 g = 6,552 Mol Dipropylmalonsäurediäthylester und 2192 g = 121,8 Mol Wasser wurden innerhalb 19,3 Stunden über das Verdampferrohr in den mit 450 kg des Katalysators IIb nach der vorstehenden Tabelle in Kugelform (Durchmesser ca. 6 mm) gefüllten Reaktor eindosiert.

Die Reaktionstemperatur betrug 298 bis 312° C.

Die Reaktionsmischung trennte sich in der Vorlage in eine wäßrige untere und eine organische obere Schicht. Die abgetrennte Phase von 1051 g wurde über ein 40 cm Füllkörperkolonne mit Porzellan-Sattelkörpern im Vakuum von 22 mbar fraktioniert destilliert.

Dabei wurden erhalten:

484 g = 2,808 Mol Dipropylessigsäureäthylester ($Kp_{22\,mbar}$ 77—78° C) Ausbeute 43,7%, bezogen auf umgesetzten Dipropylmalonester

409 g = 2,836 Mol Dipropylessigsäure ($Kp_{22\,mbar}$ 122—123° C) Ausbeute 44,3%, bezogen auf umgesetzten Dipropylmalonester

32 g = 0,131 Mol Dipropylmalonester destillierte zusammen mit der Dipropylessigsäure.

Der Umsatz an Dipropylmalonester beträgt 98% d. Th. Dipropylessigsäure und Dipropylessigsäureäthylester zusammen ergeben eine nutzbare Gesamtausbeute von 87,9%.

Der Katalysator IIa ergibt bei Verwendung gleicher Mengen gleichartige Ergebnisse.

## Beispiele 2 bis 4

Abkürzungen:

DPMDE = Dipropylmalonsäurediäthylester
DPMDM = Dipropylmalonsäuredimethylester
DPA = Dipropylessigsäure
DPAE = Dipropylessigsäureäthylester
DPAM = Dipropylessigsäuremethylester

Entsprechend Beispiel 1 mit der genannten Apparatur, jedoch mit den hochwirksamen Katalysatoren Ic bzw. Id nach der vorstehenden Tabelle wurden unter den unten angeführten Bedingungen bei praktisch vollständigem Umsatz der Ausgangsstoffe folgende Ergebnisse erhalten:

Gleichartige Ergebnisse wie im Beispiel 2 wurde bei Verwendung gleicher Mengen der Katalysatoren Ia, Ib und Ic erzielt.

| | Beispiel 2 Einsatzprodukt DPMDE | Beispiel 3 DPMDE | Beispiel 4 DPMDM |
|---|---|---|---|
| Menge in g/Mol | 4 100/16,79 | 913/3,74 | 500/2,31 |
| Wasser in g/Mol | 7 556/419,8 | 1 513/84,06 | 887/49,28 |
| Katalysator Menge (g) | Ic/450 | Ie/450 | Ie/450 |
| Korngröße in mm | Granulat 1,5—5 | Granulat 1,5—5 | Granulat 1,5—5 |
| Temperatur in °C | 220—223 | 220—222 | 229—230 |
| Dauer (Std.)*) | 55,4 | 11,28 | 6,37 |
| Umsatz in % | ≧99,9 | ≧99,9 | ≧99,9 |
| Ausbeute, bezogen auf den Umsatz, von | | | |
| DPA in % | 42,5 | 44,1 | 33,0 |
| DPAE in % | 53,9 | 48,0 | |
| DPAM in % | | | 61,3 |
| DPA + DPAE in % | 96,4 | 92,1 | |
| DPA + DPAM in % | | | 94,3 |

*) Dosierzeit

## Beispiel 5

### Herstellung des Na-Salzes aus dem Gemisch Dipropylessigsäure und Dipropylessigsäureester

Aus einem Dauerversuch zur Umsetzung von Dipropylmalonsäurediäthylester zu Dipropylessigsäure und Dipropylessigsäureäthylester wurden 400 g der organischen Phase des Reaktionsproduktes entnommen. Gaschromatographisch wurden Gehalte des Ausgangsstoffes unter 0,1, von Valeriansäure unter 0,2 Gew.-% festgestellt. Durch Kochen mit NaOH und Rücktitration wurde die Summe an Säure und Ester zu 2,381 Mol ermittelt. Diese Reaktionslösung wurde mit 324 g einer wäßrigen 29,4 Gew.-%igen NaOH (2,381 Mol NaOH) versetzt und 3 Std. am Rückfluß gekocht.

Der Gehalt an restlichem Alkali betrug 0,08% (=0,58 g NaOH).

Durch Zugabe von 2 g Dipropylessigsäure wurde neutralisiert, Äthanol abdestilliert und anschließend Wasser mit 450 ml Toluol azeotrop ausgetragen.

Der Kolbeninhalt wurde schließlich am Rotationsverdampfer im Wasserstrahl- und zuletzt im Ölpumpenvakuum bis zur Trockene eingedampft. Nach dem Trocknen bei 80°C und 20 mbar über $CaCl_2$ wurden 388 g Na-Dipropylacetat = 97,7% Ausbeute erhalten.

Die nach Ansäuern mit konz. HCl freigesetzte Dipropylessigsäure hatte eine Reinheit von 99,47%.

Die restlichen 0,53% enthielten Dipropylmalonsäure (0,016), Valeriansäure (0,28), Dipropylessigester (0,047) und drei unbekannte Verbindungen.

Beispiele 6 bis 14

Entsprechend Beispiel 1 unter Verwendung der beschriebenen Apparatur wurden in den Beispielen 6 bis 9 monosubstituierte Malonsäureester (Tabelle 1) und in den Beispielen 10 bis 12 disubstituierte Malonester bzw. ein disubstituierter Cyanessigsäureester (Tabelle 2) umgesetzt.

Im Beispiel 13 wurde Alkohol anstatt Wasser verwendet, Beispiel 14 wurde in Abwesenheit von Wasser und Alkohol durchgeführt und Beispiel 15 (vgl. Tabelle 3) verwendet einen $Al_2O_3$-Katalysator.

Tabelle 1

| | Beispiel 6<br>Einsatzprodukt<br>Äthyl-<br>malonester | | Beispiel 7<br><br>Propyl-<br>malonester | | Beispiel 8<br><br>Butyl-<br>malonester | | Beispiel 9<br><br>Phenyl-<br>malonester | |
|---|---|---|---|---|---|---|---|---|
| Menge in g/Mol | 180/0,956 | | 374/2,147 | | 200/0,925 | | 561/2,37 | |
| Wasser g/Mol | 230/12,76 | | 837/46,5 | | 200/11,11 | | 831/46,2 | |
| Katalysator/g | IIb/450 | | Ie/450 | | IIb/450 | | Ic/500 | |
| Korngröße/mm | Kugeln/6 | | Granulat/1,5—5 | | Kugeln/6 | | Granulat 1,5—5 | |
| Temperatur in °C | 285—288 | | 200—201 | | 288 | | 259—260 | |
| Reaktionszeit in Std. | 1,84 | | 6,23 | | 1,92 | | 6,25 | |
| Umsatz in % | ≧00,9 | | ≧99,9 | | 99,0 | | ≧99,9 | |
| Ausbeute, bezogen auf den Umsatz in % d. Th. von: | | | | | | | | |
| Buttersäure | 21,4 | 80,6 | | | | | | |
| Buttersäureäthylester | 59,2 | | | | | | | |
| Valeriansäure | | | 24,4 | 92,6 | | | | |
| Valeriansäuremethylester | | | 68,2 | | | | | |
| Capronsäure | | | | | 39,6 | 98,6 | | |
| Capronsäureäthylester | | | | | 59,0 | | | |
| Phenylessigsäure | | | | | | | 45,8 | 89,5 |
| Phenylessigsäureäthylester | | | | | | | 43,7 | |

Tabelle 2

| | Beispiel 10<br>Einsatzprodukt<br>Diäthylmalon-<br>ester | Beispiel 11<br><br>Phenyläthyl-<br>malonester | Beispiel 12<br><br>Dipropylcyan-<br>essigester |
|---|---|---|---|
| Menge in g/Mol | 180/0,832 | 60/0,227 | 374/1,896 |
| Wasser in g/Mol | 271/15,1 | 90/5,0 | 481/26,71 |
| Katalysator/g | IIb/450 | IIb/450 | IIb/450 |

Tabelle 2 (Fortsetzung)

| | Beispiel 10 Einsatzprodukt Diäthylmalonester | Beispiel 11 Phenyläthylmalonester | Beispiel 12 Dipropylcyanessigester |
|---|---|---|---|
| Korngröße/mm | Kugeln/6 | Kugeln/6 | Kugeln/6 |
| Temperatur in °C | 285 | 260–270 | 290 |
| Reaktionszeit in Std. | 1,94 | 1 | 4,49 |
| Umsatz in % | 91,7 | 97,4 | 82,2 |
| Ausbeute, bezogen auf Umsatz in % d. Th. | | | |
| Diäthylessigsäure | 33,0 } 96,3 | | |
| Diäthylessigsäureäthylester | 63,3 } | | |
| Phenyläthylessigsäure | | 41,6 } 77,7 | |
| Phenyläthylessigsäureäthylester | | 36,1 } | |
| Dipropylacetonitril | | | 82,0 |

Tabelle 3

| | Beispiel 13 Einsatzprodukt Dipropylmalonester | Beispiel 14 Dipropylmalonester | Beispiel 15a Dipropylmalonester | Beispiel 15b |
|---|---|---|---|---|
| Menge in g/Mol | 81,4/0,333 | 122,2/0,50 | 80/0,327 | 80/0,327 |
| Äthanol bzw. Wasser g/Mol | 138,0/3,0[1] | – | 131/7,28[2] | 127/7,06[2] |
| Katalysator/g | Ic/500 | Ic/500 | $Al_2O_3$ 250/200 | |
| Korngröße/mm | Granulat/1,5–5 | Granulat/1,5–5 | Kugeln/6 | |
| Temperatur °C | 219–220 | 239–240 | 251–253 | 351–352 |
| Reaktionszeit in Std. | 1,07 | 1,1 | 1 | 0,98 |
| Umsatz in % | ≧99,9 | 90,6 | 59,0 | 98,1 |
| Ausbeute, bezogen auf Umsatz in % d. Th. | | | | |
| Dipropylessigsäure | 7,8 } 94,0 | 2,0 } 98,7 | 8,0 } 97,2 | 49,7 } 87,5 |
| Dipropylessigsäureäthylester | 86,2 } | 96,7 } | 89,2 } | 37,8 } |

[1]) Äthanol
[2]) Wasser

**Patentansprüche**

1. Verfahren zur Herstellung substituierter Essigsäuren und/oder deren Ester bzw. substituierter Acetonitrile der Formel I

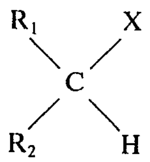

(I)

worin X —COOH, —COOY oder —CN, Y —$CH_3$ oder —$C_2H_5$, $R_1$ einen gesättigten, verzweigten oder unverzweigten aliphatischen Rest mit 1 bis 6 C-Atomen, einen Phenylrest oder einen durch Alkyl- oder Alkoxygruppen substituierter Phenylrest und $R_2$ Wasserstoff oder $R_1$ bedeuten, wobei $R_1$ und $R_2$ gleich oder verschieden sein können, aus den entsprechend substituierten Malonsäureestern oder Cyanessigsäureestern der Formel

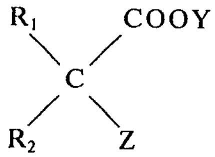

(II)

worin Z —COOY oder —CN bedeuten und Y, $R_1$ und $R_2$ die vorige Bedeutung haben, dadurch gekennzeichnet, daß man die Malon- oder Cyanessigester bei 150 bis 360° C in Gegenwart von Katalysatoren des oxidischen oder silikatischen Typs, die mindestens eines der Elemente Si oder Al enthalten, umsetzt.

2. Verfahren nach Anspruch 1, dadurch gekennzeichnet, daß die Umsetzung in Gegenwart von Wasser erfolgt.

3. Verfahren nach Anspruch 2, dadurch gekennzeichnet, daß Wassermengen von 1 Mol bis 50 Mol pro Mol Malon- oder Cyanessigester eingesetzt werden.

4. Verfahren nach Anspruch 1, dadurch gekennzeichnet, daß die Umsetzung in Gegenwart des in der Estergruppe des Malon- oder Cyanessigesters entsprechenden Alkohols durchgeführt wird.

5. Verfahren nach Anspruch 4, dadurch gekennzeichnet, daß Alkoholmengen von 1 Mol bis 50 Mol pro Mol Malon- oder Cyanessigester eingesetzt werden.

6. Verfahren nach Anspruch 1, dadurch gekennzeichnet, daß die Umsetzung in Abwesenheit von Wasser oder Alkohol erfolgt.

7. Verfahren nach Anspruch 6, dadurch gekennzeichnet, daß die Umsetzung im Temperaturbereich von 180—300° C durchgeführt wird.

8. Verfahren nach Anspruch 1, dadurch gekennzeichnet, daß in den Katalysatorsubstanzen als weitere Bestandteile eines oder mehrere der Elemente Fe, Mg, Ca, K, Na enthalten sind.

9. Verfahren nach Anspruch 1 oder 8, dadurch gekennzeichnet, daß die Katalysatoren 50—75 Gew.-% $SiO_2$ und 15—19 Gew.-% $Al_2O_3$ enthalten und Glühverluste von 15—20 Gew.-% aufweisen.

10. Verfahren nach Anspruch 1 oder 8, dadurch gekennzeichnet, daß die Katalysatoren 85—92 Gew.-% $SiO_2$ und bis 2 Gew.-% $Al_2O_3$ enthalten und Glühverluste von 6,5 bis 8 Gew.-% aufweisen oder 68—75 Gew.-% und 14—16 Gew.-% $Al_2O_3$ enthalten und Glühverluste von 6,5—8 Gew.-% aufweisen.

11. Verfahren nach Anspruch 1, dadurch gekennzeichnet, daß die Katalysatoren praktisch vollständig aus $Al_2O_3$ bestehen.

12. Verfahren nach mindestens einem der Ansprüche 1 bis 11, dadurch gekennzeichnet, daß zur Gewinnung der Produkte die organische Phase zum Produkt aufgearbeitet wird und die bei Ausführung des Verfahrens in Gegenwart von Wasser entstehende wäßrige Phase vorher abgetrennt wird.

13. Verfahren nach Anspruch 12, dadurch gekennzeichnet, daß die organische Phase in die jeweilige substituierte Essigsäure und den entsprechenden substituierten Essigsäureestern durch Destillation getrennt wird.

14. Verfahren nach Anspruch 12, dadurch gekennzeichnet, daß die gesamte organische Phase mit Gehalten der substituierten Essigsäure und des entsprechenden Esters in die Alkali- oder Erdalkali- oder Erdalkalisalze der Säure durch Erhitzen mit den jeweiligen Hydroxiden umgesetzt wird.

**Claims**

1. Process for the production of substituted acetic acids and/or esters thereof or of substitued acetonitriles of the formula I

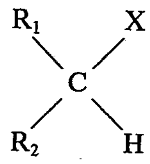

(I)

wherein X denotes —COOH, —COOY or —CN, Y denotes $-CH_3$ or $-C_2H_5$, $R_1$ denotes a saturated, branched or unbranched aliphatic residue with 1 to 6 C-atoms, a phenyl residue or a phenyl residue substituted by alkyl or alkoxy groups and $R_2$ denotes hydrogen or $R_1$, wherein $R_1$ and $R_2$ can be the same or different, from the correspondingly substituted malonic acid esters or cyanoacetic acid esters of the formula

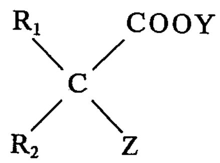

(II)

wherein Z denotes —COOY or —CN and Y, $R_1$ and $R_2$ have the previous meaning, characterised in that the malonic or cyanoacetic ester is reacted at 150 to 360°C in the presence of catalysts of oxide or silicate type which contain at least one of the elements Si or Al.

2. Process according to claim 1, characterised in that the reaction takes place in the presence of water.

3. Process according to claim 2, characterised in that water amounts of 1 mole to 50 mole per mole of malonic or cyanoacetic acid ester are employed.

4. Process according to claim 1, characterised in that the reaction is carried out in the presence of the alcohol corresponding to that in the ester group of the malonic or cyanoacetic acid ester.

5. Process according to claim 4, characterised in that amounts of alcohol of 1 mole to 50 mole per mole of malonic or cyanoacetic acid ester are employed.

6. Process according to claim 1, characterised in that the reaction takes place in the absence of water or alcohol.

7. Process according to claim 6, characterised in that the reaction is carried out in the temperature range of 180 to 300°C.

8. Process according to claim 1, characterised in that one or more of the elements Fe, Mg, Ca, K, Na are contained as further constituents in the catalyst substances.

9. Process according to claim 1 or 8, characterised in that the catalysts contain 50 to 75% by weight $SiO_2$ and 15 to 19% by weight $Al_2O_3$ and possess losses on ignition of 15 to 20% by weight.

10. Process according to claim 1 or 8, characterised in that the catalysts contain 85 to 92% by weight $SiO_2$ and up to 2% by weight $Al_2O_3$ and possess losses on ignition of 6.5 to 8% by weight, or contain 68 to 75% by weight $SiO_2$ and 14 to 16% by weight $Al_2O_3$ and possess losses on ignition of 6.5 to 8% by weight.

11. Process according to claim 1, characterised in that the catalysts consist practically completely of $Al_2O_3$.

12. Process according to at least one of claims 1 to 11, characterised in that, for obtaining the product, the organic phase is worked up to the product and the aqueous phase existing on carrying out the process in the presence of water is separated off beforehand.

13. Process according to claim 12, characterised in that the organic phase in the substituted acetic acid which is present and the correspondingly substituted acetic acid ester are separated by distillation.

14. Process according to claim 12, characterised in that the combined organic phase contents of substituted acetic acid and of the corresponding ester is converted into the alkali or alkali earth salt of the acid by heating with the appropriate hydroxides.

## Revendications

1. Procédé de préparation d'acides acétiques subtitués et/ou de leurs esters ou d'acétonitriles substitués de formule I:

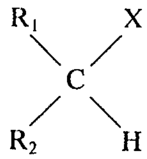

(I)

dans laquelle X représente $-COOH$, $-COOY$ ou $-CN$, Y représente $-CH_3$ ou $-C_2H_5$, $R_1$ est un radical aliphatique saturé, ramifié ou non ramifié, comportant 1 à atomes de carbone, un radical phényle ou un radical phényle substitué par des groupes alkyles ou alcoxy, et $R_2$ représente un atome d'hydrogène ou $R_1$, les symboles $R_1$ et $R_2$ pouvant être identiques ou différents, à partir d'esters de l'acide malonique ou d'esters de l'acide cyanacétique substitués de façon correspondante et répondant à la formule (II):

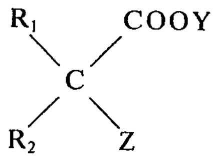

(II)

dans laquelle Z représente $-COOY$ ou $-CN$, et Y, R, et $R_2$ ont la signification ci-dessus, procédé caractérisé en ce qu'on fait réagir les esters d'acide malonique ou cyanacétique à 150 jusqu'à 360° C en présence de catalyseurs du type oxyde ou silicate, qui contiennent au moins l'un des éléments Si ou Al.

2. Procédé selon la revendication 1, caractérisé en ce que la réaction a lieu en présence d'eau.

3. Procédé selon la revendication 2, caractérisé en ce qu'on utilise des quantités d'eau comprises entre 1 mole et 50 moles par mole d'ester d'acide malonique ou cyanacétique.

4. Procédé selon la revendication 1, caractérisé en ce qu'on effectue la réaction en présence de l'alcool correspondant à celui présent dans le groupe ester de l'ester d'acide malonique ou cyanacétique.

5. Procédé selon la revendication 4, caractérisé en ce qu'on utilise des quantités d'alcool comprises entre 1 mole et 50 moles par mole d'ester de l'acide malonique ou cyanacétique.

6. Procédé selon la revendication 1, caractérisé en ce que la réaction a lieu en l'absence d'eau ou d'alcool.

7. Procédé selon la revendication 6, caractérisé en ce qu'on conduit la réaction dans un intervalle de températures allant de 180 à 300° C.

8. Procédé selon la revendication 1, caractérisé en ce que les substances à rôle de catalyseurs contiennent comme autres constituants un ou plusieurs des éléments Fe, Mg, Ca, K, Na.

9. Procédé selon la revendication 1 ou 8, caractérisé en ce que les catalyseurs contiennent 50 à 75% en poids de $SiO_2$ et 15 à 19% en poids de $Al_2O_3$ et présentent des pertes au rouge de 15 à 20% en poids.

10. Procédé selon la revendication 1 ou 8, caractérisé en ce que les catalyseurs contiennent 85 à 92% en poids de $SiO_2$ et jusqu'à 2% en poids de $Al_2O_3$ et présentent des pertes au rouge de 6,5 à 8% en poids ou contiennent 68 à 75% en poids de $SiO_2$ et 14 à 16% en poids de $Al_2O_3$ et présentent des pertes au rouge de 6,5 à 8% en poids.

11. Procédé selon la revendication 1, caractérisé en ce que les catalyseurs consistent de façon pratiquement totale en $Al_2O_3$.

12. Procédé selon l'une au moins de revendications 1 à 11, caractérisé en ce que, pour l'obtention des produits, on traite la phase organique pour obtenir le produit et en ce que, en cas de mise en œuvre du procédé en présence d'eau, on sépare au préalable la phase aqueuse résultante.

13. Procédé selon la revendication 12, caractérisé en ce qu'on sépare par distillation la phase organique en l'acide acétique substitué et en les esters d'acides acétiques substitués.

14. Procédé selon la revendication 12, caractérisé en ce que, par chauffage avec les hydroxydes respectifs, on transforme la totalité de la phase organique, contenant les acides acétiques substitués et l'ester correspondant, en obtenant les sels alcalins ou alcalino-terreux des acides.